(19) Europäisches Patentamt
European Patent Office
Office européen des brevets

(11) EP 0 806 931 B1

(12) **EUROPEAN PATENT SPECIFICATION**

(45) Date of publication and mention of the grant of the patent:
**24.07.2002 Bulletin 2002/30**

(21) Application number: **95927934.0**

(22) Date of filing: **16.08.1995**

(51) Int Cl.[7]: **A61K 6/00**

(86) International application number:
**PCT/IB95/00709**

(87) International publication number:
**WO 96/23480 (08.08.1996 Gazette 1996/36)**

(54) **COMPOSITION FOR PRIMING AND ADHERING TO TOOTH STRUCTURE**

ZUSAMMENSETZUNG ZUR GRUNDIERUNG UND ADHÄSION AN ZAHNSTRUKTUREN

COMPOSITION POUR STRUCTURES DENTAIRES UTILISABLE COMME AGENT DE LIAISON ET ADHESIF

(84) Designated Contracting States:
**AT BE CH DE DK ES FR GB IT LI NL SE**

(30) Priority: **30.01.1995 US 377555**
**30.01.1995 US 377556**

(43) Date of publication of application:
**19.11.1997 Bulletin 1997/47**

(73) Proprietor: **Dentsply GmbH**
**D-63264 Dreieich (DE)**

(72) Inventor: **BLACKWELL, Gordon**
**D-78465 Konstanz (DE)**

(74) Representative:
**Wächtershäuser, Günter, Prof. Dr.**
**Patentanwalt,**
**Tal 29**
**80331 München (DE)**

(56) References cited:
**EP-A- 0 115 410**
**EP-A- 0 161 337**
**EP-A- 0 183 027**
**WO-A-94/00096**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

The invention relates to a dental composition according to claim 1. The invention provides a composition for adhering to dental tooth surface. One or more portions of the composition may be applied and cured to form polymeric material on a cleaned tooth surface to form a treated tooth surface. A restorative material is applied to the tooth with a bond strength of at least about 12 MPa. The polymeric material has a yield strength of at least 50 MPa, an elastic modulus of at least 1,000 MPa, a resilience of at least 0.4 MPa when measured after storage in water at 37°C for 2.4 hours, and/or an initial marginal integrity between the restorative material and etched dentin or etched enamel of at least 90 percent.

It is most desirable, when filling a tooth cavity with a filling material, such as a polymerizable dental restorative, to ensure good adhesion between the tooth surrounding the cavity and the set (polymerized) filling material since there is thereby obtained a good seal between the set filling material and the tooth which prevents, or at least markedly inhibits, ingress of mouth fluids and bacteria into the filled cavity and thus prevents further decay or loss of the filling material. In order to achieve good adhesion between the filler material and the tooth enamel, it has been recommended to provide a primer or adhesive bonding layer intermediate the filling material and surfaces of a prepared tooth.

The prior art does not disclose a method of adhering polymerizable acrylate containing restoratives to dentin by applying a priming adhesive composition from only one container to provide improved retention, acceptable marginal integrity and a bond strength of at least 12 MPa as is provided by the present invention.

Bowen in U.S. Patent 3,785,832 discloses Dental Primer varnish. Pluddemann in U.S Patent 3,884,886 discloses Cationic Unsaturated Amine-Functional Silane Coupling Agents. Lee, Jr. et al in U.S. Patent 4,107,845 discloses Dental Adhesive Composites. Yamauchi et al in U.S. Patent 4,182,035 discloses Adhesive Compositions for the Hard Tissues of the Human Body. Omura et al. in U.S. Patent 4,499,251 discloses Adhesive Compositions. Billington in U. S. Patent 4,514,342 discloses Polyethylenically Unsaturated Monophosphates. Omura et al in U.S. Patent 4,515,930 discloses Highly Water-Resistant Adhesive. Martin in U.S. Patent 4,525,256 discloses Photopolymerizable Composition Including Catalyst Comprising Diketone Plus.

Omura et al. in U.S. Patent 4,537,940 discloses Adhesive Compositions. Bunker in U.S. Patent 4,540,722 discloses Dentin and Enamel Adhesive. Bunker in U.S. Patent 4,544,467 discloses Light-Curable Dentin ad Enamel Adhesive. Aasen in U.S. Patent 4,553,941 discloses Acetal and Hemiacetal Dentin and Enamel Adhesive Primers. U. S. Patent 4,589,756 relates to similar aromatic based compositions employed in dentistry. Asmussen et al. in U.S. Patent 4,593,054 disclose Adhesion Promoting Agent, Process for its preparation and use thereof on Collageneous Material. Janda in U.S. Janda in U.S. Patent 4,636,533 discloses Photopolymerizable Adhesion Promoting Dental Composition. Janda in U.S. Patent 4,640,936 discloses Photopolymerizable Phosphate-Containing Adhesive Promoting Dental Composition. James in U.S. Patent 4,645,456 discloses Adhesive Composition for Tooth Enamel. Bunker in U.S. Patent 4,669,983 discloses Dentin and Enamel Adhesive. Bunker in U.S. Patent 4,670,576 discloses Polymerizable Phosphorus Esters. Aasen in U.S. Patent 4,719,149 discloses Method for Priming Hard Tissue. Blackwell et al in U.S. Patent 4,816,495 discloses Biologically Compatible Adhesive Visible Light Curable Compositions. Bunker in U.S. Patent 4,855,475 discloses (Meth)Acrylic Esters of Phosphoric Acid Ester Dihalides. Montgomery in U.S. Patent 4,863,993 discloses Surface Priming Composition for Proteinaceous Substrates; Method of Making and Using Same. Engelbrecht in U.S. Patent 4,872,936 teaches dental cement mixtures containing polymerizable unsaturated monomers and/or oligomers and/or prepolymers containing acid groups and/or their reactive acid-derivative groups. Aasen et al in U.S. Patent 4,880,660 discloses Method for Priming Hard Tissue. Montgomery in U.S. Patent 4,913,939 discloses Method of Making and Using Surface Priming Composition for Proteinaceous Substrates. Bunker in U.S. Patent 4,929,746 discloses Dentin and Enamel Adhesive. Muggee et al in U.S. Patent 4,945,006 discloses Low Odor Adhesive Compositions and Bonding Method Employing Same. Horn et al in U.S. Patent 4,948,366 discloses Adhesive Bond Strength Control for Orthodontic Brackets. Haas in U.S. Patent 4,948,367 discloses Orthodontic Accessories and Method of Applying the Same. Huang et al in U.S. Patent 4,966,934 discloses Biological Compatible Adhesive Containing a Phosphorous Adhesion Promoter and Accelerator. Omura et al in U.S. Patent 5,064,495 discloses Method of Adhesion with a Mercapto Group Containing Adhesive. Omura et al in U.S. Patent 5,085,726 discloses Method of Adhesion with a Sulfide Group Containing Adhesive. Eppinger et al in U.S. Patent 5,089,051 discloses Adhesion-Promoting Dental Composition. Omura in U.S. Patent 5,091,441 discloses Dental Composition. Orlowski in U.S. Patent 5,141,436 discloses Method of Bonding Article to Teeth Employing a Light Curable Primer. Bunker in U.S. Patent 5,177,121 discloses Dentin and Enamel Adhesive. Kawashima et al. in U.S. Patent 5,186,783 discloses Method of Bonding with Adhesive Composition Containing a Thiocarboxylic Acid Compound. Blackwell in U.S. Patent 5,218,070 discloses Dental/Medical Composition and Use. Imai et al. in U.S. Patent 5,252,629 discloses Adhesives for Dentin. Kawashima et al. in U.S. Patent 5,254,198 discloses Method of Bonding a Metal or Alloy Utilizing a Polymerizable Thiocarboxylic Acid or a Derivative Thereof. Oxman et al. in U.S. Patent 5,256,447 discloses Adhesive Composition and Method. Ikemura et al. in U.S. Patent 5,264,513 discloses Primer Composition. Wong in U.S. Patent 5,295,824 discloses Plastic Bracket with Adhesive Primer Layer and Methods of Making. Betush in U.S. Patent 5,295,825 discloses Control System

for Dental Handpieces. Bunker in U. S. Patent 5,304,585 discloses Dentin and Enamel Adhesive.

Dental primers and adhesives in accordance with the invention have unexpectedly superior adhesion to dentin, enamel, cavity liner, bonding materials and filling materials.

It is an object of the invention to provide a dental composition useful in a method of adhering a restorative material to a dental tooth by applying to the tooth a liquid composition comprising including at least 50 percent by weight of a volatile solvent, at least 15 percent by weight of one or more polymerizable acrylate compounds and an effective amount of a photoinitiator, at least a portion of the polymerizable compounds being multifunctional polymerizable compounds having at least three acrylate moieties, to form a polymeric material which adheres to dentin with a bond strength of at least 12 MPa. The polymeric material has a yield strength of at least 50 MPa, an elastic modulus of at least 1,000 MPa, a resilience of at least 0.4 MPa and/or a marginal integrity of at least 90 percent with a restorative material.

It is an object of the invention to provide a dental composition which can be used in a method of adhering material to a tooth surface by sequentially including applying portions of a liquid priming/adhesive composition to a cleaned tooth surface and curing them to form the polymeric material having a yield strength of at least 50 MPa, an elastic modulus of at least 1,000 MPa, a resilience of at least 0.4 MPa and/or a marginal integrity of at least 90 percent with a restorative material. Clinical retention of restorative material as used herein refers to adhesive retention by bonding a restorative material to a natural living tooth in a patient's mouth effectively without undercutting the tooth.

Acceptable marginal integrity as used herein means marginal integrity which is at least 95 percent acceptable according to the Ryge criteria after one year as being alpha or bravo in accordance with Clinical Criteria by Gunnar Ryge, Inter. Dent. J, 1980; 30(4) pages 347-358 particularly at pages 348 and 349; and Cvar et al Criteria for the Clinical Evaluation of Dental Restorative Materials, U.S. Department of Health, Education and Welfare, U.S. Public Health Service Pub. No. 790-244, U.S. Government Printing Office, 1971. Microleakage at the margin is effectively eliminated by applying restoratives with acceptable marginal integrity.

Unless otherwise stated herein elastic modulus, yield strength and resilience modulus are measured according to the method discussed herein under the heading "Measurement of physical properties".

Volatile organic solvent(s) as used herein refers to organic solvent(s) which are substantially more volatile than water at 23°C.

Water miscible volatile solvent(s) as used herein refers to solvent(s) which are miscible with water, and when mixed with water effectively increase the vapor pressure of the water bring about to a substantial degree of increase in vaporization of water.

Acrylate as used herein refers to unsaturated polymerizable compounds within the general formula:

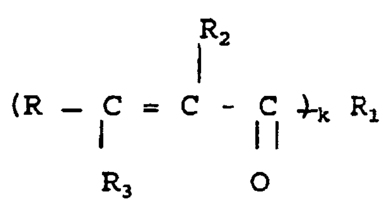

Wherein, k is an integer from 1 to 8,
R is hydrogen or methyl,
$R_1$ is an alkyl having from 1 to 20 carbon atoms,
$R_2$ is an alkyl having from 1 to 8 carbon atoms, and
$R_3$ is an alkyl having from 1 to 12 carbon atoms.

Polymerizable compound as used herein refers to monomers and/or oligomers. Acrylates are preferred polymerizable compounds.

Phosphates as used herein does not include pyrophosphates.

Bond strength in units of MPa as used herein unless otherwise indicated refers to bond strength is measured as follows. Uncontaminated, caries free, extracted human teeth without significant anatomical alterations, defects or restorations were cleaned and disinfected by soaking in 1% sodium hypo chlorite solution for 18 to 24 hours, rinsed with water and are then stored at from 1 to 8 C in 1% sodium chloride in water (saline solution) until used within six months. The wet teeth are then sanded flat by hand using wet 300 grit silicon carbide paper to expose an area of dentine at a plane just below the original interface between the enamel and the dentin, and this area of dentine is polished by hand with wet 600 grit silicon carbide paper. The teeth are kept wet in water until used within from 1 to 12 hours.

Viscosity as referred to herein is determined by a Brookfield Model LVT viscosity measuring apparatus using a number 2 spindle at 12 RPM at 25°C.

Preferred viscosity at 23°C of liquid priming adhesive compositions in accordance with the invention is less than 400 centipoise (cpi), more preferably less than 300 cpi and most preferably less than 200 cpi.

The dentine surface is dried lightly with a paper tissue, and the priming/adhesive solution of the invention applied in a thin layer using a dental operatory brush with bristles having a length of about 5 mm. The solution is allowed to stand on the dentine surface for 10 seconds (unless otherwise noted) and the remaining solvent is evaporated by blowing the tooth gently with a stream of dry oil free air. The layer of primer/adhesive remaining is light cured by irradiating it for ten seconds (unless otherwise noted) with light from a dental light curing unit having a minimum output of 350 milliwatt/square centimeter in the 400 to 500 mm wavelength range (Prismetics Lite, light curing unit, L.D. Caulk is used). A portion of plastic straw of 5mm internal diameter and about 4 mm long is placed end on to the prepared surface and filled with a light curing dental filling material (TPH, Dentsply Caulk, unless otherwise noted). Finally the filling material is cured by irradiating with light from the dental light for forty seconds.

The prepared samples are stored for 24 hours in water at 37°C before being thermocycled 500 times (unless otherwise noted) between 5°C and 55°C with a dwell time in each bath of 20 seconds. The thermocycled samples are left in water at 37°C overnight before being tested in shear using a Zwick universal testing machine model 145501 with the load cell set for a maximum load of 500 Newtons, and operating at a crosshead speed of 1 mm per minute using a 2 mm diameter cylindrical chisel.

The chisel has a tip point formed at the lower end by grinding and polishing a planar surface across the end of the cylinder at a 45 degree angle to the central axis of the cylinder. The tip point is formed at the intersection of a planar surface with the lower end of the chisel. In test position the tip point of the chisel is applied against the composite. Each tooth is vertically mounted in plastic for the test.

## SUMMARY OF THE INVENTION

The invention is a composition useful for a method for adhering a restorative material to a dental tooth by applying a liquid composition to at least a portion of said tooth to form a treated surface. A first portion of photopolymerizable liquid priming adhesive dental composition is applied to a tooth surface. After leaving the first portion of the liquid composition on the tooth surface undisturbed for at least 15 seconds on the tooth surface air is conveyed against the first portion of the liquid. The light is impinged on the first portion of the liquid composition whereby at least a portion of the first portion of the liquid composition polymerizes to form a first coating on the tooth. A second portion of the liquid composition is applied to the first coating, and air is substantially immediately conveyed to the second portion of the liquid composition. Light is then impinged on the first and second portions of the liquid composition whereby at least a portion of the second portion of the composition polymerizes to form a treated tooth surface adapted to retain a restorative material at a clinical retention rate of at least 98 percent with acceptable marginal integrity. Composition of the invention includes at least 50 percent by weight of a volatile organic solvent, at least 15 percent by weight of one or more polymerizable acrylate compounds and an effective amount of a photoinitiator. At least a portion of the polymerizable compounds are multifunctional polymerizable compounds having at least three acrylate moieties. The polymerizable compounds are substantially soluble in the solvent. The composition is adapted to form a polymeric material which adheres to dentin with a bond strength of at least 12 MPa. The cured resin has a yield strength of at least 50 MPa, an elastic modulus of at least 1,000 MPa, a resilience of at least 0.4 MPa when measured after storage in water at 37°C for 24 hours, and/or an initial marginal integrity between the restorative material and etched dentin or etched enamel of at least 90.

## DETAILED DESCRIPTION OF THE INVENTION

A method using the dental composition of the invention comprises conditioning a tooth surface, including applying a first portion of a liquid priming adhesive composition to a cleaned tooth surface and curing the first portion of liquid primer adhesive composition to form a primed tooth surface. Optionally a second portion of the liquid priming adhesive composition is applied to the primed tooth surface and cured. The polymeric material has a yield strength of at least 50 MPa, an elastic modulus of at least 1,000 MPa, a resilience of at least 0.4 MPa and/or provides an initial marginal integrity of at least 90 percent.

A polymerizable restorative composition is bonded to the tooth with a bond strength of at least about 12 MPa. Priming adhesive compositions useful in accordance with the invention preferably include in order of increasing preference at least 50, 60, 70 or 80 percent by weight of a volatile solvent and at least 15 percent by weight of a polymerizable compound. The present invention reduces microleakage by infiltration at the interface of the restorative and the tooth.

A method employing a preferred embodiment of the invention comprises treating a natural tooth by applying

a first portion of photopolymerizable liquid primer/adhesive dental composition to tooth surface to form a first coating. After leaving the first portion of the liquid composition on the tooth surface undisturbed for at least 15 seconds on the surface, a second portion of the liquid composition is applied to the first coating, and air is conveyed to the second portion of the liquid composition.

Then light is impinged on the first and second portions of the liquid composition so that at least a portion of the second portion of said composition polymerizes to form a treated tooth surface adapted to retain a restorative material at a clinical retention rate of at least 98 percent.

In accordance with a preferred embodiment, the dental composition of the invention may be used in a method of adhering a restorative material to a dental tooth by applying a dental composition which includes at least 50 percent by weight of a volatile solvent, at least 15 percent by weight of one or more polymerizable acrylate compounds and an effective amount of a photoinitiator. At least a portion of the polymerizable compounds are multifunctional polymerizable compounds having at least three acrylate moieties. The polymerizable compounds are substantially soluble in the solvent. The composition is adapted to form a polymeric material which adheres to dentin with a bond strength of at least 12 MPa.

Preferably the composition includes in order of increasing preference at least 2, 2.5, 3, 3.5, 4, 5, 6, or 7 percent by weight of the multifunctional polymerizable compounds. Preferably the solvent is dimethyl ketone or methyl ethyl ketone and the bond strength is at least 15 MPa. Preferably the composition comprises at least 75 percent by weight of said solvent. Preferably at least a portion of the multifunctional polymerizable compounds are phosphate esters.

Preferably at least a portion of the multifunctional compounds have a chemical structure within the scope of the general formula:

$$(R)_n\text{-}Z\text{-}(A)_m$$

wherein each R independently is an acrylate containing moiety, Z is an organic moiety, each A is independently is a phosphate, n is an integer greater than 2, m is an integer of 1 or more. Preferably at least a portion of the polymerizable compounds are acids and the acids comprise at least 2 percent by weight of the composition. Preferably at least a portion of the polymerizable compounds are acid esters.

In accordance with a preferred embodiment, the dental composition of the invention may be used in a method of adhering a restorative material to a dental tooth by applying a liquid composition to at least a portion of the tooth to form a treated surface. The composition includes at least 50 percent by weight of a volatile solvent, at least 15 percent by weight of one or more polymerizable acrylate compounds and an effective amount of a photoinitiator. At least a portion of the polymerizable compounds are multifunctional polymerizable compounds having at least three acrylate moieties. The polymerizable compounds are substantially soluble in the solvent. The composition is adapted to form a polymeric material which is adapted to adhere to dentin with a bond strength of at least 12 MPa. Restorative material is then affixed to at least a portion of the treated surface with a bond strength of at least about 12 MPa.

Acid-etching of enamel may be done but is not necessary. To fill deep tooth cavities it is preferred to cover the dentine closest to the pulp of the tooth with a hard-setting calcium hydroxide liner (such as DYCAL, sold by Dentsply International Inc.) leaving the rest of the cavity floor and walls free for chemical bonding with a dental restorative such as Dyract, sold by Dentsply International Inc.

Preferred volatile solvents include, ethanol, methanol, isopropanol, dimethyl ketone, ethylmethyl ketone, and mixture of these.

Preferred monomers for use in primer adhesive compositions in accordance with the inventor have a solubility in water of less than about 5%, and more preferably have a solubility in water of less than 1%. Exemplary monomers include triethylene glycol dimethacrylate, tetraethyleneglycol dimethacrylate, glycerol-1,2-dimethacrylate, glycerol-1,3-dimethacrylate, the reaction product of butanediol diglycidyl ester and methacrylic acid, tetrahydrofurfural methacrylate, methacryloxyethyl maleic ester, methacryloxyethyl succinate, urethane dimethacrylate, Bis-GMA, Ethoxylated bisphenol-A dimethacrylate, bisphenol-A dimethacrylate, and mixtures thereof. Monomers having a solubility in water higher than 5% are less preferred. Monomer having a solubility in water less than about 1% are more preferred. Highly water soluble monomers such as hydroxyethyl methacrylate and hydroxypropyl methacrylate tend to provide lower adhesion and are less suitable for use in compositions of the invention.

A volatile solvent is removed after application of the primer of the dentine surface. The monomer is preferably less volatile than the solvent.

In use, compositions of the invention are applied to a clean dry dentine surface, and the solvent is evaporated, for example, by application of a gentle stream of air. The layer of resin remaining is preferably cured by exposing it to light from a dental curing lamp. The composite filler formation is then applied by exposing it to light from a dental curing lamp and cured.

Prior art dentine adhesive systems giving adhesion higher than 12 MPa have required separate applications

of a primer composition and an adhesive composition. The combined primer/adhesive composition of the present invention achieves high bond strength adhesion levels with application of one liquid.

As the free radical-polymerizable monomer or prepolymer to be employed in this invention, use may be made of any monomer, dimer, trimer, or other oligomer of the type that is usable in dental applications. Thus, the polymerizable monomer portion of the present adhesive composition generally comprises one or more monofunctional or polyfunctional ethylenically unsaturated monomers or prepolymers, e.g., dimers, trimers, and other oligomers, or mixtures or copolymers thereof, based on acrylic or methacrylic or itaconic acid, or derivatives thereof, including their esters which can be polymerized by free radical initiation. These materials include, but are not limited to acrylic and methacrylic acid, itaconic acid and the like, acrylic or methacrylic or itaconic acid esters of monohydric or polyhydric alkanols or polyhydric alcohols containing at least one phenyl group. Examples of such compound include monovinylmethacrylates, e.g., methylmethacrylate, ethyl acrylate, propyl methacrylate, hydroxyethylmethyacrylate, hydroxypropylmethacrylate, diethylene glycol acrylate, triethylene glycol acrylate, the monoester of trimellitic acid with hydroxyethyl methacrylate, hydroxypropyl itaconate and the like, esters of aliphatic polyhydric alcohols, such as for example, the di- and polyacrylates, the di- and polymethacrylates, and the di- and polyitaconates of alkylene glycols, alkoxylene glycols, alicyclic glycols and higher polyols, such as ethylene glycol, triethylene glycol, tetraethylene glycol, tetramethylene glycol, trimethylolethane, trimethylolpropane, pentaerythritol, dipentaerythritol, tripentaerythritol, and the like, or mixtures of these with each other or with their partially esterified analogs, and their prepolymers, such compound or mixture optionally having free hydroxyl content. Typical compounds of this type, include but are not limited to, trimethylolpropane triacrylate, trimethylolethane triacrylate, trimethylolpropane trimethacrylate, trimethylolethane trimethacrylate, tetramethylene glycol dimethacrylate, ethylene glycol dimethacrylate, triethylene glycol dimethacrylate, tetraethylene glycol diacrylate, tetraethylene glycol dimethacrylate, pentaerythritol diacrylate, pentaerythritol triacrylate, pentaerythritol tetraacrylate, dipentaerythritol diacrylate, dipentaerythritol triacrylate, dipentaerythritol tetraacrylate, dipentaerythritol pentaacrylate, dipentaerythritol hexacrylate, tripentaerythritol octaacrylate, pentaerythritol dimethacrylate, pentaerythritol trimethacrylate, dipentaerythritol dimethacrylate, glycerin trimethacrylate, ethylene glycol dimethacrylate, butanediol dimethacrylate, trimethylolpropane trimethacrylate, tetramethylolmethane tetramethacrylate,bisphenol-A dimethacrylate, bisphenol-A diglycidyl methacrylate, 2,2,'-bis-(4-methacryloxyethoxyphenyl) propane and so on.

Also included among the polymerizable monomers which may be used are the vinyl urethane or urethane-acrylate prepolymers which are well known in the art. These prepolymers are polymerizable by free radical initiation and may be prepared, for example, by reacting an organic diisocyanate or an isocyanate-terminated urethane prepolymer with an ethylenically unsaturated monomer which is reactive with the diisocyanate or urethane prepolymer. These polymers also may be prepared by reacting a hydroxyl-containing material, such as a polyol or a hydroxyl-terminated urethane prepolymer with an ethylenically unsaturated monomer which is reactive with the polyol or hydroxyl-terminated urethane. The urethane prepolymers, which may be linear or branched, carry isocyanate end groups and generally are prepared by reacting a compound having hydroxyl functionality with a molar excess of diisocyanate.

Any of a wide variety of diisocyanates may be used to prepare the isocyanate-terminated urethane prepolymer including aliphatic, cycloaliphatic, heterocyclic, and aromatic diisocyanates, and combinations of these. Examples include, but are not limited to, 2,4-tolylene diisocyanate, 2,6-tolylene diisocyanate, 1,4-phenylene diisocyanate, 2,2,4-trimethyl hexamethylene diisocyanate, hexamethylene diisocyanate, 1,4-naphthalene diisocyanate, 1,5-naphthalene diisocyanate, 4,4,'-diphenylmethane diisocyanate, p,p, '-diphenyl diisocyanate, butylene-1,4-diisocyanate, ethylene diisocyanate, trimethylene diisocyanate, tetramethylene-1,4-diisocyanate, butylene-2,3-diisocyanate, cyclohexylene-1,2-diisocyanate, methylene-bis-(4-phenyl-isocyanate), diphenyl-3,3,'-dimethyl-4,4,'-diisocyanate, xylylene diisocyanate, cyclohexane-1,4-diisocyanate, l-methoxyphenyl-2,4-diisocyanate and the like, and mixtures thereof.

A wide variety of compounds having hydroxyl functionality may be used to form the isocyanate-terminated urethane prepolymers. For example, diols of the structure

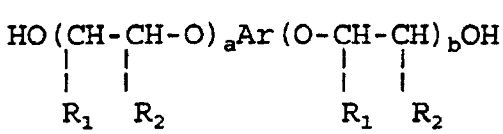

may be used, where R1 and R2 are hydrogen atoms or alkyl groups, e.g., methyl, and Ar is a divalent aromatic group in which each free valency is on an aromatic carbon atom, and where a and b, independently, may be zero or an integer. Other suitable hydroxyl containing compounds include diols and polyols such as ethylene glycol, propylene glycol, triethylene glycol, tetramethylene glycol, trimethylolpropane, pentaerythritol, dipentaerythritol, and the like, or esters of acrylic acid, methacrylic acid or itaconic acid or the like with aliphatic polyhydric alcohols. Among the more preferred hydroxyl containing compounds are the esters of acrylic or methacrylic acid and a hydroxyalkanol of at least two carbon

atoms such as hydroxyethyl acrylate, hydroxyethyl methacrylate, hydroxypropyl acrylate, hydroxypropyl methacrylate, hydroxyisopropyl methacrylate, and the like.

Formation of the isocyanate terminated urethane prepolymers may be assisted by the use of a catalyst known in the art to assist polyurethane formation, for example, tertiary amines and metal salts, e.g., tin salts, titanium salts and the like.

To form the vinyl urethane or urethane-acrylate prepolymer starting materials, an isocyanate-terminated urethane prepolymer or a diisocyanate is reacted with an ethylenically unsaturated compound having hydroxyl functionality. These compounds include for example, esters of acrylic acid, methacrylic acid or itaconic acid with aliphatic polyhydric alcohols, such as hydroxyethyl acrylate, hydroxypropyl methacrylate or the like. The resulting vinyl urethanes are well known in the art and are described for example, in U.S. Pat. No. 3,629,187 to Waller, U.S. Pat. No. 3,759,809 to Carlick et al, U.S. Pat. No. 3,709,866 to Waller and U.S. Pat. No. 4,459,193 to Ratcliffe et al.

Formation of the vinyl urethane prepolymers may be assisted by the use of the same catalysts noted above, namely, tertiary amines and metal salts.

The foregoing list of polymerizable ethylenically unsaturated monomers and prepolymers is intended to be exemplary only, and other known polymerizable materials can be used in compositions of this invention.

In accordance with a preferred embodiment of the invention two or more ethylenically unsaturated compounds are included in dental treatment compositions.

In a preferred embodiment of the invention the polymerizable monomer is liquid at temperatures from about 20° C to about 25° C.

Preferred monomers are TEGDMA, glyceryl dimethacrylate, polyethylene glycol dimethacrylate, trimethylolpropane, trimethacrylate, UDMA, R5-62-1, EBPDMA, and ethylene glycol dimethacrylate.

Preferred solvents are ethanol, 2-propanol, and dimethyl ketone.

A preferred bonding composition in accordance with a preferred embodiment of the invention includes about 5 percent by weight of PENTA; about 13.2 percent by weight of urethane diacrylate; 0.59 percent by weight of Bisphenol-4-dimethacrylate; 0.2 percent by weight of camphorquinone (CQ); 0.6 percent by weight of EDAB; 0.1 percent by weight of BHT; from 0 to 0.4 percent of an organic fluoride salt, and 78 to about 81 percent by weight of dimethyl ketone.

A preferred etchant includes 36 percent by weight of $H_3PO_4$ and 64 percent by weight of water.

Exemplary acrylic monomers for use in compositions of the invention include: 1,4-butanediol dimethacrylate (BDEM); glyceryl dimethacrylate (GlyDM); hydroxyethyl methacrylate (HEMA); triethyleneglycol dimethacrylate (TGD); tetrahydrofuran dimethacrylate (THFMA).

Preferably a restorative composition is applied to a tooth surface treated in accordance with the invention, and photocured to form a restorative filling having a clinical retention rate after one year of at least 98 percent. Preferably prior to applying liquid primer/adhesive to a tooth surface the surface of smear layer of the tooth is removed, for example by acid etching by applying dilute or concentrated acid(s), or at neutral pH by applying EDTA. Thus, the photopolymerizable dental restorative composition is applied to the treated tooth surface, light is then impinged on the restorative composition whereby at least a portion of the restorative composition polymerizes to form a cured restorative material adhered with a high bond strength to the treated tooth surface.

This bond strength is greater than a comparative bond strength of a bond between the restorative material and a tooth formed essentially by applying a first portion of the liquid composition to the treated tooth surface. Then impinging light on the first portion of the liquid composition. A photopolymerizable dental restorative composition is then applied to the treated tooth surface. Light is then impinged on the restorative composition whereby at least a portion of the restorative composition polymerizes to form a cured restorative material adhered with a comparative bond strength to the treated tooth surface.

In accordance with a preferred embodiment, the dental composition of the invention may be used in a method for clinical retention of restorative material by a natural tooth by applying a portion of photopolymerizable liquid priming adhesive dental composition to tooth surface. After leaving the first portion of the liquid composition on the tooth surface undisturbed for at least 15 seconds on the surface, air is conveyed (forced or directed) against the first portion of the liquid on the surface. Light is then impinged on the first portion of the liquid composition whereby a portion of the first portion of the liquid composition polymerizes to form a first coating on the tooth. A second portion of the liquid composition is applied to the first coating. Substantially immediately thereafter air is conveyed to the second portion of the liquid composition. Light is then impinged (irradiated) to the first and second portions of the liquid composition whereby at least a portion of the second portion of the composition polymerizes to form a treated tooth surface adapted to retain a restorative material at a clinical retention rate of at least 98 percent.

Preferably the first portion of the liquid composition is left on the surface undisturbed for at least 15 seconds. Preferably the solvent is miscible with water and when mixed with water substantially lowers the effective vapor pressure of the water bringing about a substantial degree of increased vaporization of the water in-situ at the treated surface. Preferably the liquid dental composition has a viscosity of less than 400 centipoise. Preferably the liquid dental composition includes oligomer having a weight average molecular weight greater than 600.

Preferably before applying the first of the liquid composition at least a portion of the tooth is treated by steps of:

(1) applying a dilute acid to the tooth surface to form an etched tooth surface,
(2) rinsing the dilute acid from the etched tooth surface to form a rinsed etched surface, and
(3) partially drying the rinsed etched surface to form a wetted etched surface.

Preferably the first portion of the liquid composition remains undisturbed on the surface for at least 20 seconds, and has a viscosity of less than 300 centipoise and includes at least 70% of a water miscible volatile solvent. Preferably the liquid composition includes at least 2% of an oligomer having a weight average molecular weight greater than 650.

In accordance with a preferred embodiment, the dental composition of the invention may be used in a method of adhering a restorative material to a natural tooth by applying a first portion of photopolymerizable liquid primer/adhesive dental composition to tooth surface. After leaving the first portion of the liquid composition on the tooth surface undisturbed for at least 15 seconds on. the surface, air is conveyed against the first portion of the liquid on the surface. Light is then impinged on the first portion of the liquid composition whereby a portion of the first portion of the liquid composition polymerizes to form a first coating on the tooth. A second portion of the liquid composition is applied to the first coating, and substantially immediately conveying air to the second portion of the liquid composition. Light is impinged on to the first and second portions of the liquid composition whereby at least a portion of the second portion of the composition polymerizes to form a treated tooth surface adapted to retain a restorative material at a clinical retention rate of at least 98 percent. Preferably liquid composition remains on the surface substantial undisturbed for at least 20 seconds, has a viscosity of less than 300 centipoise, includes at least 70% of a water miscible volatile solvent, and at least 2% of an oligomer having a weight average molecular weight greater than 650.

In the wet dentin techniques dentin surface is exposed and ground flat with 600 grit paper. Dentin is rinsed with clean water prior to bonding. Primer or primer/adhesive is then applied. A cotton pellet is used to remove any excess moisture on the dentin surface leaving a small amount of residual moisture on the immediate surface of the dentin.

Total Etching refers to preparation of the tooth surfaces by applying ten percent phosphoric acid for 10 to 15 seconds, then rinsing the acid off thoroughly for 10 to 15 seconds.

Shear dentin bond strengths of dentin through the Primer/Adhesive composition of the invention to Dyract Restorative Material are in excess of 18 MPa and have at least 98 percent retention of restorative.

Shear dentin bond strengths achieved with primer/adhesive of the invention with TPH restorative on wet unetched dentin is 19 MPa. The shear bond strength for TPH restorative are approximately 37% higher for primer/adhesive of the invention than with ProBond of the prior art.

In accordance with a preferred embodiment, the dental composition of the invention may be used in a method of adhering a restorative material to a dental tooth by applying a dental composition which includes at least 50 percent by weight of a volatile solvent, at least 15 percent by weight of one or more polymerizable acrylate compounds and an effective amount of a photoinitiator. At least a portion of the polymerizable compounds are multifunctional polymerizable compounds having at least three acrylate moieties. The polymerizable compounds are substantially soluble in the solvent. The composition is adapted to form a polymeric material which adheres to dentin with a bond strength of at least 12 MPa.

Having generally described the invention, a more complete understanding can be obtained with reference to certain specific examples, which are included for purposes of illustration only. It should be understood that the invention is not limited to the specific details of the Examples.

FIRST COMPARATIVE EXAMPLE

A commercially available primer composition is made by stirring 6 percent by weight of PENTA, 30 percent by weight of HEMA, 63.9 percent by weight of 95% ethanol; and 0.1 percent by weight of BHT. The primer composition is applied to a cleaned tooth surface, solvent is blown away, and then Dyract, a polymerizable acrylate containing restorative is applied which forms a bond strength of 6.3 MPa.

SECOND COMPARATIVE EXAMPLE

The procedure of the First Comparative Example is followed except that after removal of solvent, a commercially available adhesive composition is applied and cured. The adhesive composition is made by stirring 24.90 percent by weight of triethylene glycol dimethacrylate, 9.24 percent by weight of bisphenol-A-dimethacrylate, 0.03 percent by weight of butylated hydroxy toluene, 2.0 percent by weight of morpholino ethyl methacrylate, 0.12 percent by weight of lithium P-toluenesulfinate, 0.24 percent by weight of camphorquinone, 53.32 percent by weight of urethane diacrylate, 4.55 percent by weight of DPEPAP, and 5.6 percent by weight of HEMA. The restorative forms a bond having

bond strength of 10 Mpa.

EXAMPLE 1A

A) Priming Adhesive Composition

A priming and adhesive polymerizable composition in accordance with the invention is formed by stirring 3.13 grams of 7,7,9,63,65 hexamethyl-4,13, 60,69-tetra-oxo-3,14,19,24,29,34,39,44,49,54,59,70-dodecanaoxa-5,12,61,68-tetra-azadoheptacontane-1,72 diyl-dimethacrylate, (also known as urethane dimethacrylate resin); 5.00 grams of 2,2,6-6 tetra acryloxyloxymethyl-4, 8 dioxa-9-oxo-11-undecyl phosphoric acid, also known as dipentaerythritol pentacrylate phosphoric acid ester (PENTA); 11.07 grams of 2,7,7,9, 15-pentamethyl-4,13-dioxo-3,14-dioma-5,12-diaza hexadecane 1,1 diyl dimethacrylate; also known as urethane dimethacrylate (UDMA) ; 0.1 grams of phenol-2,6-bis-(1,1-dimethethyl-4-methyl), also known as butylated hydroxytoluene (BHT); 0.2 grams of bicyclo [2.2.1] heptane-2, 3-dione-1,7,7-trimethyl, also known as camphorquinone; 0.60 grams of 4-Ethyl dimethyl aminobenzoate (DMABE), 0.59 grams of isopropylidenbis-4-phenoxymethacrylate also known as bis-phenol-A-dimethacrylate (BPADMA) and 79.31 grams of dimethyl ketone.

B) Priming a tooth surface

Extracted human teeth are treated in 1% sodium hypochlorite for 18 to 24 hours, washed with water, and stored in distilled water in a refrigerator at about 4°C until needed. The teeth are mechanically wet sanded successively with 320/600 grit carborundum paper until the dentin is exposed. Each tooth sample is then etched with 36% phosphoric acid for 20 seconds, rinsed with water for 15 seconds, and then air dried to remove surface water. The priming adhesive composition (made as described in Example 1A section A)is then applied with a brush and left to stand for 30 seconds, dried with oil-free air for five seconds, and cured for 10 seconds with a Prismetics™Lite light curing unit. Prisma®TPH™ restorative is placed in a cylindrical plastic matrix with a 5.0 mm inside diameter, set on the treated dentin and cured by irradiating with the curing unit for 40 seconds. The specimens are stored in distilled water for approximately 24 hours at 37°C. Each specimen is mounted vertically in a plastic cylinder with self cure polymethyl methacrylate so that the dentin surface is parallel to the Instron needle; and then debonded on a Zwick Universal Testing Machine with a crosshead speed of 1 mm/minute. The priming adhesive has an elastic modulus of 1641 MPa, a yield strength of 60.7 MPa and a resilience modulus of 1.1 MPa. The restorative bonds to the treated tooth surface with a bond strength of 13.9 MPa, and marginal integrity as shown in Tables 1 and 1A.

THIRD COMPARATIVE EXAMPLE

A) Priming Adhesive Composition

Dyract PSA (TM) priming and adhesive sold by Dentsply G.m.b.H. polymerizable composition in a single bottle is used in this example.

B) Priming a tooth surface

Extracted human teeth are treated in 1% sodium hypochlorite for 18 to 24 hours, washed with water, and stored in distilled water in a refrigerator at about 4°C until needed. The teeth are mechanically wet sanded successively with 320/600 grit carborundum paper until the dentin is exposed. Each tooth sample is then etched with 36% phosphoric acid for 20 seconds, rinsed with water for 15 seconds, and then air dried to remove surface water. The priming adhesive composition (Dyract PSA priming adhesive) is then applied with a brush and left to stand for 30 seconds, dried with oil-free air for five seconds, and cured for 10 seconds with a Prismetics™ Lite light curing unit. Prisma®TPH™ restorative is placed in a cylindrical plastic matrix with a 5.0 mm inside diameter, set on the treated dentin and cured by irradiating with the curing unit for 40 seconds. The specimens are stored in distilled water for approximately 24 hours at 37°C. Each specimen is mounted vertically in a plastic cylinder with self cure polymethyl methacrylate so that the dentin surface is parallel to the Instron needle; and then debonded on a Zwick Universal Testing Machine with a crosshead speed of 1 mm/minute. The priming adhesive has an elastic modulus of 612 MPa, a yield strength of 16.8 MPa and a resilience modulus of 0.23 MPa. The restorative bonds to the treated tooth surface with a bond strength of 12.0 MPa, and marginal integrity as shown in Tables 1 and 1A.

Marginal leakage in class V cavities

Method:

Cylindrical class V cavities in human teeth are filled with the priming adhesive of Example 1A, or the Third Comparative Example or a reference adhesive plus TPH. Syntac and ProBond are used as reference materials. Both the enamel and total etch technique are carried out and the results are shown below in Tables 1 and 1A. The etched specimens are etched for 30 seconds using 36% phosphoric acid. Each specimen is thermocycled (1500 times) and thereafter analyzed for marginal breakdown.

Table 1:

| **Enamel etch technique** | | |
|---|---|---|
| Adhesive system | percent perfect margin after thermocycling | |
| | in enamel | in dentine |
| Example 1A | 96.2 (3.5) | 72.0(7.7) |
| Syntac | 94.1 (4.9) | 54.2(29.2) |
| ProBond | 83.8 (12.8) | 48.9(25.0) |
| Dyract PSA (Third Comparative Example) | 87.7(7.4) | 66.2(18.6) |

Table 1A:

| **Total etch technique** | | |
|---|---|---|
| Adhesive system | percent perfect margin after thermocycling | |
| | in enamel | in dentine |
| Example 1A | 96.1(3.0) | 91.8(5.4) |
| Dyract PSA (Third Comparative Example) | 92.2(6.3) | 72.8(16.8) |

**Marginal leakage in class II cavities (MOD)**

MOD cavities are prepared and subsequently etched (enamel and dentine) with 36% phosphoric acid for 15 seconds. In each case, one of the approximal boxes ended in the enamel region while the other was extended into the dentine area. The cavity walls are covered with either of the adhesive systems(the composition of Example 1A, Dyract PSA or ProBond) and then filled with TPH composite filling material. During placement of the restoration and the stress test the specimens are subjected to pressure simulating the dentinal liquid. The entire cavity is filled with two increments of composite and cured from the occlusal direction. The stress is applied to the restored teeth by 1.2 million chewing cycles and thermocycling 3000 times.
Thereafter the specimens are investigated for marginal integrity by means of SEM technique.

Table 2:

| Total Etch Technique | | |
|---|---|---|
| Adhesive system | percent perfect margin initially | after stress application |
| Example 1A | 91.2 (3.8) | 63.2(4.8) |
| Dyract PSA (Third Comparative Example) | 81.5 (9.4) | 53.0(12.1) |
| ProBond | 85.3 (4.3) | 59.4(8.0) |

ProBond is sold by Dentsply International. Scotch Bond Multi-Purpose (TM) system is sold by 3M. ProBond and Scotch Bond are both sold with separate bottles of primer and adhesive solutions. In use, the primer is first applied and blown dry, but not cured. A separate adhesive is next applied and cured, followed by the composite material.

Syntac (TM) system is sold by Vivadent as two solutions which are applied sequentially with intermediate

drying, but neither layer is cured. Finally an adhesive resin is applied before application of the composite material.

Kerr's OptiBond (TM) system has a primer which is applied and light cured, followed by application from a second bottle of a layer of adhesive and light curing before application of the composite material.

Clearfil Liner Bond II (TM) sold by Kuraray includes three separate bottles. Solutions from two of the bottles are mixed, and the mixture applied to a tooth surface and blown dry but not cured. A third solution is then applied to the tooth surface and cured before application of composite material.

Denthesive II (TM) is sold by Kulzer, two separate solutions which are mixed, applied to the tooth surface and blown dry, but not light cured. A third solution is applied to the tooth surface and then cured before application of composite material.

Gluma (TM) is sold by Bayer. The dentine is first cleaned with a specially provided solution, washed with water and blown dry. A primer is then applied and blown dried, and finally a solution called a "sealer" is applied, the composite material is placed on top, and the sealer and composite are cured together.

Table 3

Physical properties of the adhesive resin part of various adhesive systems

| Material | After storage one day in water at 37°C | | | After storage one month in water at 37°C | | | Percent decrease in property | | | Number of separate solutions |
|---|---|---|---|---|---|---|---|---|---|---|
| | E (MPa) | Y (MPa) | R (MPa) | E (MPa) | Y (MPa) | R (MPa) | E (MPa) | Y (MPa) | R (MPa) | |
| Example 1A | 1641 | 60.7 | 1.1 | 1187 | 39.3 | 0.7 | 28 | 35 | 36 | 1 |
| Dyract PSA (Third Comparative Example) | 612 | 16.8 | 0.23 | 187 | 5.1 | 0.1 | 70 | 70 | 57 | 1 |
| ProBond | 573 | 18.5 | 0.29 | 267 | 7.4 | 0.1 | 53 | 60 | 65 | 2 |
| Scotchbond Multi-Purpose | 2180 | 84.3 | 1.63 | 1862 | 61.9 | 1.03 | 15 | 27 | 37 | 2 |
| Gluma | 1793 | 69.1 | 1.33 | 1905 | 69.1 | 1.26 | 15 | 0 | 5 | 3 |
| Syntac/ Heliobond | 1992 | 76.1 | 1.46 | 1920 | 69.3 | 1.26 | 4 | 9 | 14 | 3 |
| OptiBond | 1714 | 60.2 | 1.06 | 1722 | 60 | 1.05 | 0 | 0 | 1 | 2 |
| Liner Bond II | 2042 | 79.0 | 1.53 | 1306 | 39.7 | 0.61 | 36 | 50 | 60 | 3 |
| Denthesive II | 1384 | 51.2 | 0.95 | 1533 | 53.2 | 0.92 | -11 | -4 | 3 | 3 |

Measurement of physical properties

Test specimens for each material are prepared by filling the uncured resin (after removal of solvent if necessary) into cylindrical molds 4mm diameter and 6mm long, and curing the resin for 40 seconds from each end by irradiation with light from a dental curing lamp. The hardened specimens are then removed from the molds and stored under water at 37°C for the required length of time. The specimens are then tested in compression along the axis of the cylinder using a Zwick Universal Testing Machine set to a crosshead speed of 1mm per minute.
Throughout this disclosure elastic modulus E is derived from the following equation.

$$E = \frac{\text{stress}}{\text{stress}} = \frac{F \text{ X } l}{e \text{ X } A}$$

where F is the applied force, e is the change in length, l is the original length, A is the cross sectional area of the material under stress.
Throughout this disclosure yield strength is derived from the equation

$$Y = \frac{P}{A}$$

Where P is the force at the proportional limit, and A is the cross sectional are of the specimen under test.
Throughout this disclosure resilience modulus R is calculated from the equation

$$R = \frac{Y^2}{2E}$$

where E an Y are the elastic modulus and yield strength as defined above.
While present embodiments of the invention and methods of practicing the same have been illustrated and described, it will be recognized by those skilled in the art that this invention may be otherwise variously embodied and practiced within the scope of the following claims.

**Claims**

**1.** A dental composition which comprises:

(a) at least 50 percent by weight of a volatile organic solvent,
(b) at least 15 percent by weight of polymerizable acrylate compounds adapted to form an elastomer when polymerized and being substantially soluble in said solvent, said polymerizable compounds comprising

(b1) at least a portion of multifunctional polymerizable compounds having at least three acrylate moieties and one phosphate moiety and
(b2) a further polymerizable compound having at least two acrylate moieties and a gram molecular weight greater than 200, and

(c) an effective amount of a photoinitiator,

whereby said composition is adapted to bond restorative dental material to tooth dentin by forming a polymeric material adhering to dentin with a bond strength of at least 12 MPa.

**2.** The composition according to claim 1, wherein said polymeric material provides an initial marginal integrity after etching the enamel of at least 90 percent.

**3.** The composition according to claim 1 or 2, wherein said polymeric material has an elastic modulus of at least 1000 MPa.

**4.** The composition according to any one of claims 1 to 3, wherein said polymeric material has a yield strength of at least 50 MPa.

**5.** The composition according to any one of claims 1 to 4, wherein said polymeric material has a resilience modulus of at least 0.4 MPa.

**6.** The composition of any one of claims 1 to 5, wherein said composition comprises at least 3 percent by weight of said multifunctional polymerizable compounds.

**7.** The composition of any one of claims 1 to 5, wherein said composition comprises at least 4 percent by weight of said multifunctional polymerizable compounds.

**8.** The composition of any one of claims 1 to 5, wherein said composition comprises at least 7 percent by weight of said multifunctional polymerizable compounds.

**9.** The composition of any one of claims 1 to 8, wherein said solvent is dimethyl ketone or methyl ethyl ketone and said bond strength is at least 15 MPa.

**10.** The composition of any one of claims 1 to 9, wherein said composition comprises at least 75 percent by weight of said solvent.

**11.** The composition of any one of claims 1 to 10, wherein at least a portion of said multifunctional polymerizable compounds are phosphate esters.

**12.** The composition of any one of claims 1 to 11, wherein at least a portion of said multifunctional polymerizable compounds have a chemical structure within the scope of the general formula:

$$(R)_n\text{-}Z\text{-}(A)_m$$

wherein each R independently is an acrylate containing moiety, Z is an organic moiety, each A independently is an acid or acid ester, n is an integer greater than 2, m is an integer of 1 or more.

**13.** The composition of claim 1 to 12 wherein said solvent is dimethyl ketone, at least a portion of said polymerizable compounds are acids and said acids comprise at least 2 percent by weight of said composition.

## Patentansprüche

**1.** Dentalzusammensetzung, die folgendes umfasst:

(a) mindestens 50 Gewichtsprozent eines flüchtigen organischen Lösungsmittels,
(b) mindestens 15 Gewichtsprozent an polymerisierbaren Acrylatverbindungen, die dazu eingerichtet sind ein Elastomer zu bilden, wenn sie polymerisiert werden, und die im Wesentlichen in dem Lösungsmittel löslich sind, wobei die polymerisierbaren Verbindungen

(b1) mindestens einen Anteil an mehrfunktionellen polymerisierbaren Verbindungen, die mindestens drei Acrylatgruppen und eine Phosphatgruppe und
(b2) eine weitere polymerisierbare Verbindung, die mindestens zwei Acrylatgruppen und ein Molekulargewicht von mehr als 200 g/mol umfassen und

(c) eine wirksame Menge eines Fotoinitiators,

wobei die Zusammensetzung dazu eingerichtet ist ein dentales Restaurationsmaterial an Zahndentin zu binden, indem ein polymeres Material gebildet wird, das an dem Dentin mit einer Haftstärke von mindestens 12 MPa haftet.

**2.** Zusammensetzung nach Anspruch 1, wobei das polymere Material nach einem Ätzen des Schmelzes eine Randanfangsintegrität oder Randanfangsunversehrtheit von mindestens 90 Prozent bereitstellt.

**3.** Zusammensetzung nach Anspruch 1 oder 2, wobei das polymere Material einen Elastizitätsmodul von mindestens 1000 MPa aufweist.

4. Zusammensetzung nach einem der Ansprüche 1 bis 3, wobei das polymere Material eine Streckfestigkeit von mindestens 50 MPa aufweist.

5. Zusammensetzung nach einem der Ansprüche 1 bis 4, wobei das polymere Material einen Verformungsmodul von mindestens 0,4 MPa aufweist.

6. Zusammensetzung nach einem der Ansprüche 1 bis 5, wobei die Zusammensetzung mindestens 3 Gewichtsprozent von den mehrfunktionellen polymerisierbaren Verbindungen umfasst.

7. Zusammensetzung nach einem der Ansprüche 1 bis 5, wobei die Zusammensetzung mindestens 4 Gewichtsprozent von den mehrfunktionellen polymerisierbaren Verbindungen umfasst.

8. Zusammensetzung nach einem der Ansprüche 1 bis 5, wobei die Zusammensetzung mindestens 7 Gewichtsprozent von den mehrfunktionellen polymerisierbaren Verbindungen umfasst.

9. Zusammensetzung nach einem der Ansprüche 1 bis 8, wobei das Lösungsmittel Dimethylketon oder Ethylmethylketon ist und die Bindungsstärke mindestens 15 MPa beträgt.

10. Zusammensetzung nach einem der Ansprüche 1 bis 9, wobei die Zusammensetzung mindestens 75 Gewichtsprozent von dem Lösungsmittel umfasst.

11. Zusammensetzung nach einem der Ansprüche 1 bis 10, wobei mindestens ein Teil der mehrfunktionellen polymeriserbaren Verbindungen Phosphatester sind.

12. Zusammensetzung nach einem der Ansprüche 1 bis 11, wobei mindestens ein Teil der mehrfunktionellen polymeriserbaren Verbindungen eine chemische Struktur innerhalb des Umfangs der folgenden allgemeinen Formel aufweisen:

$$(R)_n\text{-}Z\text{-}(A)_m$$

wobei jedes R unabhängig für eine ein Acrylat enthaltende Gruppe steht, Z für eine organische Gruppe steht, jedes A unabhängig für eine Säure oder einen Säureester steht, n für eine ganze Zahl steht, die größer als 2 ist, und m für eine ganze Zahl von 1 oder größer als 1 steht.

13. Die Zusammensetzung nach einem der Ansprüche 1 bis 12, wobei das Lösungsmittel Dimethylketon ist, mindestens ein Teil der polymerisierbaren Verbindungen Säuren sind und die Säuren zu mindestens 2 Gewichtsprozent in der Zusammensetzung enthalten sind.

## Revendications

1. Composition dentaire qui comprend :

(a) au moins 50 % en poids d'un solvant organique volatil,
(b) au moins 15 % en poids de composés consistant en acrylates polymérisables aptes à former un élastomère une fois polymérisés et substantiellement solubles dans ledit solvant, lesdits composés polymérisables comprenant

(b1) au moins une partie constituée de composés polymérisables multifonctionnels ayant au moins trois groupements acrylate et un groupement phosphate, et
(b2) un composé polymérisable supplémentaire ayant au moins deux groupements acrylate et un poids moléculaire en grammes supérieur à 200, et

(c) une quantité efficace d'un photo-initiateur,

ladite composition étant ainsi apte à former un matériau dentaire adhésif de restauration avec la dentine des dents en formant une matière polymère adhérant à la dentine avec une force adhésive d'au moins 12 MPa.

**2.** Composition suivant la revendication 1, dans laquelle la matière polymère engendre une intégrité marginale initiale après une attaque de l'émail d'au moins 90 %.

**3.** Composition suivant la revendication 1 ou 2, dans laquelle la matière polymère a un module élastique d'au moins 1000 MPa.

**4.** Composition suivant l'une quelconque des revendications 1 à 3, dans laquelle la matière polymère a une limite d'élasticité d'au moins 50 MPa.

**5.** Composition suivant l'une quelconque des revendications 1 à 4, dans laquelle la matière polymère a un module de résilience d'au moins 0,4 MPa.

**6.** Composition suivant l'une quelconque des revendications 1 à 5, qui comprend au moins 3 % en poids desdits composés polymérisables multifonctionnels.

**7.** Composition suivant l'une quelconque des revendications 1 à 5, qui comprend au moins 4 % en poids des composés polymérisables multifonctionnels.

**8.** Composition suivant l'une quelconque des revendications 1 à 5, qui comprend au moins 7 % en poids des composés polymérisables multifonctionnels.

**9.** Composition suivant l'une quelconque des revendications 1 à 8, dans laquelle le solvant est la diméthylcétone ou la méthyléthylcétone et la force adhésive est égale à au moins 15 MPa.

**10.** Composition suivant l'une quelconque des revendications 1 à 9, qui comprend au moins 75 % en poids du solvant.

**11.** Composition suivant l'une quelconque des revendications 1 à 10, dans laquelle au moins une partie des composés polymérisables multifonctionnels est constituée d'esters du type phosphate.

**12.** Composition suivant l'une quelconque des revendications 1 à 11, dans laquelle au moins une partie des composés polymérisables multifonctionnels a une structure chimique répondant à la formule générale :

$$(R)_n\text{-}Z\text{-}(A)_m$$

dans laquelle chaque groupe R représente, indépendamment, un groupement à fonction acrylate, Z représente un groupement organique, chaque groupe A représente, indépendamment, un acide ou ester d'acide, n représente un nombre entier supérieur à 2 et m représente un nombre entier égal ou supérieur à 1.

**13.** Composition suivant les revendications 1 à 12, dans laquelle le solvant consiste en diméthylcétone, au moins une partie des composés polymérisables consiste en acides et ces acides constituent au moins 2 % en poids de ladite composition.